# EUROPEAN PATENT APPLICATION

(11) **EP 1 360 940 A1**
(43) Date of publication of application: **12.11.2003**
(21) Application number: 03251669.2
(22) Date of filing: 18.03.2003
(51) Int. Cl.: A61B 19/04, A61L 31/16, A41D 19/00

(54) **Anti-microbial elastomeric flexible article, such as a glove, and manufacturing method**

(30) Priority: 02.05.2002 US 138370
(71) Applicant: Chou, Belle L., Union City, CA 94587 (US)
(72) Inventor: Chou, Belle L., Union City, CA 94587 (US)
(74) Representative: Evens, Paul Jonathan

(57) **Abstract**

An anti-microbial elastomeric flexible article is disclosed which includes a thin layer (10) of an anti-microbial preparation disposed on an inside surface of the elastomeric flexible article (12) such as a glove. For example, the preparation may include an acidic substance. Preferably, the preparation further includes a buffer. Preferably, the preparation is in a substantially dehydrated form and is activated by exposure to perspiration during use.

## Description

The present invention relates to elastomeric flexible articles, for example, gloves or the like.

Disposable gloves, for example, disposable examination gloves, have been widely used as a protective measure to insulate hands from objects handled by the glove wearer. To allow ease in handling objects, disposable gloves typically are made of thin and elastic material to minimize the space between the skin and the glove. Due to poor air circulation resulting from a tight fit, hand perspiration can be a common problem among glove wearers. Prolonged wearing of disposable gloves can cause a moist environment on the surface of the hand that allows viruses, bacteria, yeast, fungus and other infectious agents to grow and multiply. Itchiness and irritation can be a frequent result of wearing disposable examination gloves for extended periods.

Powders are commonly used on the inner surface of disposable gloves to alleviate perspiration and to make donning, wearing and removal of gloves easier. However, there are several disadvantages that can be associated with powders. Continuous perspiration can easily overwhelm the thin layer of powder that is commonly attached to the surface of the glove. This is especially the case when continuous and frequent wearing of gloves is required. For example, dentists may continuously wear gloves during a dental surgical procedure for up to 40 minutes or more. In addition, hand washing is necessary after the use of powdered gloves. Frequent hand washing to remove powders is inconvenient and may also cause excessive dryness of the skin.

Aside from gloves intended primarily to protect the hand during performing of tasks, special-purpose gloves have been constructed that are intended primarily to surround the hand with lotions to condition and sooth the skin. Typically, these gloves contain multiple layers in the glove design. For example, these moisturizing gloves can contain a middle layer saturated with lotion and a porous inner layer that allows the lotion to pass through and contact the user's skin. See U.S. Pat. No. 5,614,202. Other examples of moisturizing gloves can contain an inner lining made of a lotion absorbent material. By impregnating the lotion into the absorbent material, the lotion can condition the hands while the gloves are worn. See U.S. Pat. Nos. 4,186,445 or 4,185,330.

Compared to single layer disposable gloves, the complex design of multiple layer gloves can make their production far more costly. More importantly, the thickness of the layers and the complicated structures of the gloves can hinder hand flexibility and sensitivity when the glove wearer tries to pick up and manipulate objects. Such multiple layer designs can be suitable for moisturizing hands, but are generally not suitable for use in manipulating objects, especially for professions that require performing of fine tasks with precision.

In order to treat or protect skin, some people apply lotions, creams, or powders onto their skin, for example, onto their hands. Some people apply such lotions, creams, or powders immediately before donning disposable gloves to perform fine tasks. See, for example, International Patent Application No. WO94/12115. However, separate application of the preparations by users remains unsatisfactory in a variety of ways.

For example, the extra step of separately applying such preparations by a glove user is inconvenient. The extra step also tends to be omitted, due to forgetfulness and/or inconvenience. Further, manual application by the user can result in application of inconsistent quantities of the preparations or significantly uneven distributions of the preparations. Applying too much or uneven distributions of a preparation can lead to discomfort or unfamiliar inhibition of hand dexterity or sensitivity. Applying too little of a preparation can lead to insufficient beneficial effect. Further, use of powders within gloves has drawbacks, as has been discussed; such drawbacks are especially pronounced if the amount or distribution of the powder is not or cannot be well controlled, for example due inappropriate reliance on a mere ordinary glove user to apply the powder. Even aside from other drawbacks of using powder, if an ordinary glove user applies too much powder or poorly distributed powder into a glove, the powder can actually start falling out of the glove during use, which can be unacceptable.

Still further, conventional skin preparations for gloves may be incapable of prolonged effectiveness within gloves, in the presence of accumulating perspiration and other substances that can overwhelm the preparations. Still further, conventional skin preparations for gloves may contain substances that are undesirable to some users for some applications, for example, substances that are unfamiliar to users (for example, antibacterial agents that do not occur naturally) or substances that are suspected of being harmful (for example, conventional antiperspirants).

### SUMMARY

There exists a need for elastomeric flexible articles, for example, gloves or the like, for example, disposable examination gloves or the like, that apply helpful substances to skin during use.

According to an embodiment of the present invention, an article includes: a disposable protective glove, the disposable protective glove having an interior surface; and a preparation disposed on the interior surface of the disposable protective glove, wherein the preparation includes an anti-microbial substance, and wherein the preparation includes a buffer that helps resist change in pH during wearing of the disposable protective glove.

According to another embodiment of the present invention, an article includes: a disposable protective glove, the disposable protective glove having an interior surface; and a preparation disposed, during factory production of the article, on the interior surface of the disposable protective glove, wherein the preparation includes an anti-bacterial substance, and wherein the preparation includes a buffer that helps resist change in pH during wearing of the disposable protective glove.

According to another embodiment of the present invention, a protective glove includes: only a single layer of a fluid-impermeable flexible material having an inner surface and forming a cavity to receive a hand; and an anti-microbial preparation coated on the inner surface, wherein no aloe vera is coated on the inner surface, and wherein the anti-microbial preparation was factory pre-coated onto the inner surface.

According to other embodiments of the present invention, there is a method for making any glove according to any embodiment of the present invention.

### DESCRIPTION OF THE DRAWINGS

Features, aspects and advantages of some embodiments of the present invention will become better understood with reference to the accompanying drawings, which are not to be considered limitations in the scope of the invention, but are merely illustrative.
FIG. 1 shows a front perspective view of one embodiment of the present invention, in which the elastomeric flexible article is a glove.
FIG. 2 is a sectional view of the elastomeric flexible article shown in FIG 1.
FIG. 3 is a flow diagram that illustrates a process, according to some embodiments of the present invention, for making a coated elastomeric article.
FIG. 4 is a flow diagram that illustrates a method for manufacturing treated gloves, for example using spraying, according to an embodiment of the present invention.
FIG. 5 is a flow diagram that illustrates a method, according to an embodiment of the present invention, for manufacturing treated gloves that is integrated with, and includes, the manufacturing of the underlying preparation-free gloves themselves.

### DETAILED DESCRIPTION OF SPECIFIC EMBODIMENTS

The description above and below and the drawings of the present document discuss one or more currently preferred embodiment(s) and also describe some exemplary optional feature(s) and alternative embodiment(s). The description and drawings are for the purpose of illustration and not limitation. Section titles are terse and are for convenience and not limitation.

According to an embodiment of the present invention, there is provided an elastomeric flexible article. According to an embodiment of the present invention, there is provided a method of manufacturing.

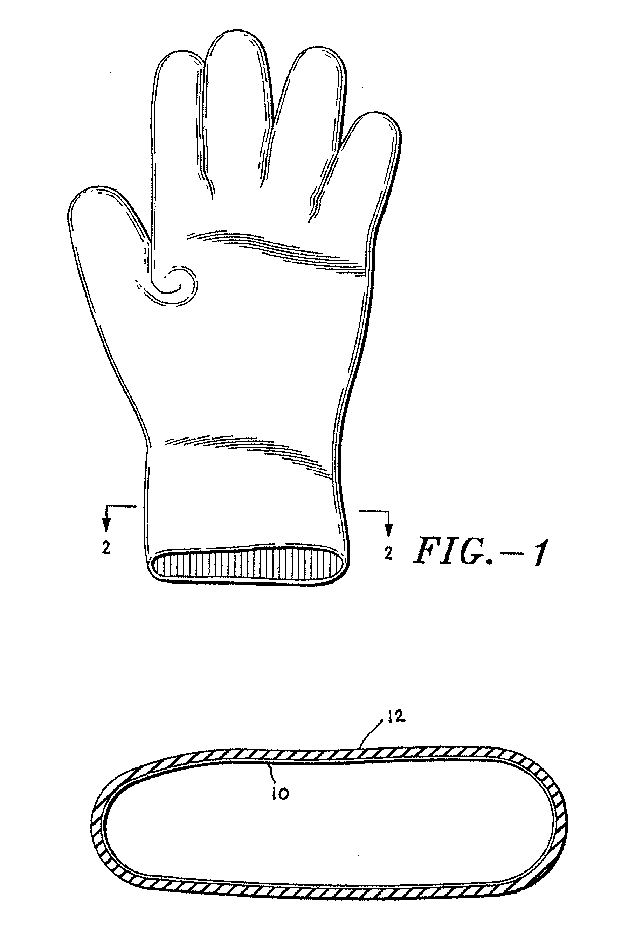

As illustrated in FIGS. 1 and 2, an elastomeric flexible article according to some embodiments of the present invention has a preparation 10 on the inner surface of the elastomeric flexible article. The flexible article is shown as a glove in FIGS. 1 and 2, but other forms of articles may also be used. During use, the coated inner surface of the elastomeric flexible article is in contact with human skin and intermingles with perspiration from the skin and, due to presence of the preparation 10, has a property of being anti-bacterial, anti-fungal, or anti-viral, or a combination thereof.

For example, the coated inner surface in contact with human skin is more anti-bacterial, anti-fungal, and/or anti-viral than it would be if it were bare and not coated with the preparation 10. The preparation 10 is an anti-bacterial, anti-fungal, and/or anti-viral preparation. Preferably, the preparation 10 is an anti-bacterial, anti-fungal, and/or anti-viral preparation that has been dehydrated. The preparation 10 may be attached onto the inner surface of the elastomeric flexible article due to an affiliation force provided by dehydration of the preparation 10.

In some embodiments of the present invention, the elastomeric flexible article is a protective glove. For example, the glove may be embodied as a disposable examination glove made of a single layer of fluid-impermeable material, which is simple and convenient to use and allows the user to wear the glove and to perform fine tasks with precision. For example, the protective glove may be embodied as a glove without any layer of porous material overlying the hand, e.g., without any layer of porous material. For example, the protective glove may be embodied to be without any absorbent material overlying the hand, e.g., without any absorbent material.

The protective glove can be made of various materials to form a layer 12. To those of ordinary skill in the art, resinous materials such as vinyl or the like or polymer materials such as acrylonitrile or the like are common choices. Three commonly used materials for making disposable gloves are natural rubber latex, acrylonitrile, and polyvinyl chloride, although any other elastomeric material may also be used. Still other materials, for example, polyurethane, chloroprene, neoprene, butadiene, or the like, or any elastomeric material known to those with ordinary skill in the art may also be used. Preferably, the preparation is evenly distributed on the interior surface of the disposable protective glove.

According to one embodiment of the present invention, during use of the glove, the environment encountered by the hand within the glove is acidic, due to presence of the preparation. For example, the preparation may be an acidic preparation that has been dried onto the inner surface of the glove, and perspiration from the hand moistens the dried acidic preparation. The acidic preparation may be a mixture that includes an acidic solution, and the mixture may, but need not, itself be a solution. Preferably, the preparation contains a buffer, to help maintain the pH and stabilize pH drift. Whether or not the preparation was dried onto the inner surface of the glove, the preparation during use is acidic in the embodiment. Preferably, the pH of the preparation during use is lower than about 6, for example, between about 3.8 to about 6, or, more preferably, between about 4.5 to about 6, or between about 5 to about 5.8. Preferably, the preparation is formulated to maintain pH within the desired range even after some prolonged use, e.g., even after some prolonged perspiration.

One embodiment of the invention is formulated such that, when exposed to increased moisture over time, pH within a desired range is still successfully obtained. For example, pH within an above-discussed desired range is still successfully obtained using, for example, the following test scenario or its like. According to a test scenario, the preparation (for example, the dried preparation) upon a glove's surface is exposed to a moist environment. For example, the glove is placed into a chamber having high humidity for some duration of time. The humidity moistens the preparation. If pH is then measured on the moist preparation, the measurement result is an acidic pH, for example, of between 3.8 and 6, or, more preferably, between 4.5 to 6 or between 5 to 5.8. For example, the high humidity may be about 100% humidity; the temperature in the chamber may be at least 30 degrees Celsius; the duration of time may be at least about 30 minutes; the pressure in the chamber may be at least standard pressure (i.e., at least one bar) through the duration; pH may be measured using any conventional system, for example, a conventional probe-type or litmus-paper-type system; and the glove may be arranged inside out, with its coated surface facing outward, for example, supported by a hand-shaped mechanical form onto which the inside-out glove is "worn". Other test scenarios, for example, similar scenarios, may also be used.

Prolonged contact between latex and oil-based substances can adversely affect durability and flexibility of the latex. Most commercially available lotions typically contain oil-based substances. The use of such conventional lotions can substantially shorten the shelf life of a latex glove.

In one embodiment of the present invention, the elastomeric flexible article is a disposable glove, coated as discussed above, and the glove is made of natural rubber latex. In this embodiment, the preparation preferably does not contain any oil-based substances that would be conventionally detectable and that would come into contact with the glove. Optionally, the preparation also does not contain any oil-based substances that would be conventionally detectable and that would come into contact with skin during use.

Another embodiment of the present invention is a glove, coated according to any discussion in the present document, in which the underlying glove is made of acrylonitrile polymer. As mentioned above, in still other embodiments, a glove is made of still any other elastomeric material, coated according to any discussion in the present document.

The preparation may be disposed onto the elastomeric flexible article by any manner whatsoever. For example, the preparation may be disposed onto the elastomeric flexible article in dry (e.g., powder) or moist (e.g., wet mixture) form. In one embodiment of the present invention, the preparation is preferably disposed onto the elastomeric flexible article, e.g., glove, in non-powder form. Preferably, the preparation is disposed onto the elastomeric flexible article in non-dry form and then is preferably fully or at least substantially dehydrated. Preferably, the dehydration is conducted such that the preparation is dehydrated onto the elastomeric flexible article, and such that there is a force provided by the dehydration that attaches the preparation to a surface of the elastomeric flexible article. Preferably, the preparation is disposed onto the elastomeric flexible article during factory production, and not by an end buyer or end owner or end wearer of the article.

In wet form, the preparation preferably includes, as mentioned above, an acidic solution. The acidic solution has pH lower than 7. For example, the acidic solution may have pH lower than about 6. In one embodiment of the present invention, the acidic solution has pH no lower than about 3.8, for example, within the range of about 3.8 to about 6. A pH no lower than about 3.8 is believed to be less likely to irritate skin. In another embodiment of the present invention, the acidic solution has a pH no lower than about 4.5, for example, within the range of about 4.5 to about 6 or within the range of about 4.5 to about 5.8. Or, the acidic solution may have a pH no lower than about 5, for example, within the range of about 5 to about 6 or within the range of about 5 to about 5.8. Such pH no lower than about 4.5 or no lower than about 5 is believed to be less likely to have significant skin-exfoliation properties. Less exfoliation can be desirable in embodiments meant for frequent or prolonged wearing. Despite the mentioned preferred pH ranges, acidic solutions with even lower pH can nevertheless still be used if desired, for example, for intended infrequent or short-duration use, or with other ingredients in the preparation that have been selected according to conventional knowledge to mitigate or ameliorate the potential for skin irritation. Low pH, for example down to about 3.8 or even lower, may also be desired if skin exfoliation is desired.

In a preferred embodiment of the present invention, the preparation contains, as mentioned above, a buffer to help maintain the pH and stabilize pH drift. Typical buffers include and are not limited to weak inorganic acids with its conjugate base, weak organic acids with its conjugate base, amino acids and amino acid salts, for example. In one embodiment of the present invention, enough buffer will be used, according to conventional knowledge and experience, to exhibit a pH drift of, for example, about 0.15 pH unit or less. However, less or more buffer may also be used. Generally, it is well known to those skilled in the art that quartenized phosphate esters can reduce the pH drift of a solution from about 0.2 to 0.3 pH units to about 0.15. Any competent buffer can be used. Buffers are well known to those of ordinary skill in the art.

Still further, optionally, thickeners can be used in the preparation to promote more even coating. Typical thickeners used preferably are non-greasy and non-oily compounds. Exemplary polymers and thickeners are listed in the CTFA Cosmetic Ingredient Handbook, 1st Ed., J. M. Nikitakis ed., The Cosmetic, Toiletry and Fragrance Association, Washington, DC (1988) (hereafter CTFA Handbook), at pages 30, 47, 48, 67 and 97-100, incorporated herein by reference. Example thickening agents include but are not limited to polyacrylic acid, polyacrylate, polymethacrylic acid, polyacreylamide, sodium alginate, gelatin, xantham gum, acacia, aga carboxymethyl cellulose, carboxymethyl cellulose salt, polyvinyl alcohol, polyvinyl acetal, polyvinylpyrrolidone, magnesium aluminum silicate, sold under the tradename VEEGUM and available in various grades from R. T. Vanderbilt Co., Inc., Norwalk, Conn.; aluminum starch octenylsuccinate; a water-soluble polymer, like a polyvinylalcohol, a polybutene, a polyethylene glycol, or a polyethylenimine; or a gum, like xanthan gum, hydroxyethylcellulose, karaya gum, carrageenen, hydroxypropyl guar, methylcellulose, tragacanth gum, or hydroxypropylcellulose.

In some embodiments of the invention, the preparation can include other optional ingredients, for example, antiperspirants and/or skin soothing substances, or the like. Skin soothing substances include, for example, skin moisturizing substances or skin anti-irritant substances. In addition, the preparation can also include other optional ingredients, for example, glycerin, which is a water-soluble emollient and emulsion aid, preservatives, fragrances, or dyes, or the like.

Examples of antiperspirants include aluminum-zirconium tetraclorohydrex glycine, alcloxa, aluminum cloride, aluminum chlorohydrex, aluminum PCA, zirconium chlorohydrates, aluminum zirconium tetrachlorohydrates, and aluminum chlorohydrates, and the like, or any other antiperspirant, for example, antiperspirants known to those of ordinary skill in the art.

Examples of skin soothing substances include, for example, a skin moisturizing agent, especially for embodiments of the invention that are not dried onto the glove. Examples also include aloe vera, lotions, creams, and the like.

In some preferred embodiments of the present invention, the preparation does not include any antiperspirant, for example, no antiperspirant from among aluminum-zirconium tetraclorohydrex glycine, alcloxa, aluminum cloride, aluminum chlorohydrex, aluminum PCA, zirconium chlorohydrates, aluminum zirconium tetrachlorohydrates, and aluminum chlorohydrates.

In some embodiments of the present invention, the preparation does not include any skin soothing substance. In some embodiments of the present invention, the preparation does not include any aloe vera. In some embodiments of the present invention, the only skin soothing substance in the preparation is aloe vera.

The acidic solution within the preparation typically includes an organic acid, such as a hydroxycarboxylic acid, herein termed a "hydroxy acid". The acidic solution within the mixture typically includes an alpha-hydroxycarboxylic acid, herein termed an "alpha-hydroxy acid". In accordance with an embodiment of the present invention, the acid solution present typically is a hydroxycarboxylic acid, generally an alpha-hydroxycarboxylic acid, for example, glycolic acid.

The particular amount of acid included in the preparation is dependent upon the type of acid, the production method and equipment, and the intended end use for the preparation-coated glove, for example, frequent or long-duration wearing, infrequent or short-duration wearing, use primarily to deter infection, or use to deter infection and also to exfoliate skin.

In one embodiment of the present invention, the preparation contains about 0.1% to about 20% by weight of an acid, before being dry. Toward the higher end of this range, skin exfoliation abilities tends to be greater. In another, more preferred embodiment of the present invention, the preparation contains about 0.1% to about 10% by weight of an acid, before being dry. In another embodiment of the present invention, the preparation contains about 0.2% to about 2% by weight of an acid, before being dry. The acid may be a hydroxy acid, or another type. Whatever the actual concentration or type of acid used, whether explicitly listed herein or not, the invention is preferably embodied so as also to achieve the earlier-discussed desired pH values.

Generally, cosmetologists and dermatologists use high concentrations of hydroxy acids (for example, 50 to 70 percent by weight) as superficial peels, to smooth rough skin, and to remove fine lines, acne scars, age spots, irregular pigmentation, and precancerous scaly patches. Moderate concentrations of hydroxy acids have typically been seen (for example, 10 to 50 percent by weight) to help control acne by unplugging pores, and to enhance the effectiveness of Retin-A and skin bleaches. However, at these concentrations, the hydroxy acid-containing products often provide dramatic results, but the potential to irritate or burn the skin is high. At hydroxy acid concentrations of, for example, 30% by weight or more, the compositions are capable of chemically burning the skin.

Accordingly, it is helpful to balance the acidic nature of an acidic solution with the skin-irritation potential of the solution. Many acid-containing compositions, including hydroxy acid-containing compositions, often warn the user that a tingling or burning sensation may be felt after the first several applications of the composition to the skin. In accordance with some embodiments of the present invention, it is preferable to provide an elastomeric flexible article, such as a disposable glove, that minimizes or avoids the tingling or burning sensation or irritation that can be associated with chemical burns due to acids, yet provide the beneficial antibacterial, anti-fungal, antiviral effects of these acids.

The hydroxy acid, or any other organic acid, in the acid solution can be present in the free acid form, in the salt form, or as a mixture of the free acid and salt form. Often, the acid is present as a mixture of the free acid form and salt form in order to provide a solution having a pH in a desired range, such as those mentioned above. When the hydroxy acid is present in the salt form, the hydroxy acid is neutralized with a water-soluble alkali until the desired pH is achieved. The water-soluble alkali can be for example, ammonia or ammonia hydroxide, a water-soluble amine, or an alkali metal hydroxide. Preferred water-soluble alkalis include but are not limited to ammonia, ammonium hydroxide, diethanolamine, triethylamine, methylamine, triethanolamine, potassium hydroxide, sodium hydroxide, lithium hydroxide, or a primary, secondary or tertiary amine having alkyl or hydroxyalky groups containing one to three carbon atoms.

In accordance with an embodiment of the present invention, the hydroxy acid in the acidic solution may be any acid. For example, the hydroxy acid can be an aliphatic acid, e.g., glycolic acid; an aromatic acid, e.g., salicylic acid; or have aromatic and aliphatic components, e.g., mandelic acid. Exemplary hydroxy acids include the alpha-hydroxy acids, such as, but not limited to, glycolic acid, citric acid, lactic acid, tartaric acid and malic acid. These alpha-hydroxy acids are naturally-occurring acids found in fruit, and have been used in skin care and skin treatment compositions for several years. It has been theorized that glycolic acid and lactic acid are the most effective alpha-hydroxy acids, if exfoliation is desired, because these acid molecules are small and more able to penetrate skin. Hydroxycaprylic acid is a synthetic alpha-hydroxy acid that has been used in skin care compositions. Other useful alpha-hydroxy acids are, for example, mandelic acid, leucic acid, azelaic acid and ethylglycolic acid.

Beta-hydroxy acids, like salicylic acid, beta-hydroxypropionic acid and beta-hydroxybutyric acid, also are useful in the acidic solution of an embodiment of the present invention. In general, any aliphatic alpha- or beta-hydroxy acid having an aliphatic carbon chain containing two through ten carbon atoms can be used in the acidic solution. The hydroxy acid can be a monocarboxylic acid, a dicarboxylic acid or a polycarboxylic acid.

The acid in the acidic solution is not limited to hydroxy acids. Essentially any acid that is used, or can be used, in cosmetic compositions for skin can be incorporated into the present solution. The acids traditionally are organic acids.

Specific acids that can be incorporated into the acidic solution include, but are not limited to, glucuronic acid, glutamic acid, allantoin galacturonic acid, allantoin glycyrrhetinic acid, allantoin polygalacturonic acid, animal collagen amino acids, animal elastin amino acids, animal keratin amino acids, aspartic acid, folic acid, hyaluronic acid, linoleic acid, linolenic acid, orotic acid, palmitoyl animal collagen amino acids, ribonucleic acid, silk amino acids, uric acid, urocanic acid, dilinoleic acid, trilinoleic acid, alanine, 6-aminocaproic acid, arginine, asparagine, carbocysteine, cysteine, cystine, glycine, histidine, hydroxyproline, isoleucine, leucine, lysine, methionine, phenylalanine, proline, serine, threonine, tryptophan, tyrosine, biotin, o-cresotic acid, glycyrrhetinic acid, glycyrrihizic acid, lanolin acid, and mixtures thereof. Other organic acids, either carboxylic acids, amino acids, or an organic acid having an ionizable hydrogenated atom, also can be incorporated into the acidic solution.

In one preferred embodiment, the anti-bacterial solution includes an acid that exists naturally in a plant, preferably in an edible plant. Preferably, the anti-bacterial solution includes substantially no antibacterial substance other than the acid that exists naturally in a plant, or in an edible plant.

In one embodiment of the present invention, the acidic solution contains malic acid. Optionally, the acidic solution contains no other acid other than malic acid. Malic acid is a natural organic acid found in food sources such as green apples, currants and other fruits. It has a very strong but pleasant sour taste. Malic acid is commonly used in the winemaking process to control wine acidity and tartness. It is also used in fruit juice, soda water and soft drinks. Malic acid is also used as an acidulant, flavoring agent and color stabilizer in food processing. It is known to be one of the gentler food acids and is considered to be a safe substance for ingestion or use on the skin. Preferably, the mixture containing the malic acid solution also includes a buffer.

The acidic glove according to an embodiment of the present invention retains the characteristic of a disposable examination glove without any externally visible structural modification, and is easy and convenient to use. The affiliation between the acidic mixture (for example, a buffered malic acid solution) and the glove surface may be through a force provided by dehydration. Such affiliation is loosened when perspiration dissolves the dehydrated acidic pH solution. The longer a glove is worn, the more likely the hand will perspire, and consequently more acidic solution will be dissolved and disassociated from the glove surface, and be applied to the hand. The acidity of the solution can then condition hand skin and prevent microorganisms from growing under the wet condition.

In one embodiment, a solution of malic acid with a pH of about 5.5 is used to coat the gloves. Malic acid solution is distributed on the inner surface of the glove at a thickness of about 0.01 millimeter. Preferably, the distribution of the Malic acid is substantially even and uniform. Preferably, the association between malic acid and the surface is achieved at least in part due to a non-covalent force provided through dehydration.

In one specific embodiment, the preparation is formulated, for example, as follows (percentages are by weight): 0.5% Aloe Vera 200X concentrated powder; 0.04% Sodium Bensoate (99.5% concentrate, as a preservative); 0.04% Potassium Sorbate (98.0% concentrate, as a preservative); 0.02% Carboxymethylcellulose Sodium (90% concentrate, as a thickener); 1.0% Citric Acid (99.0% concentrate, acid to make pH 5.5); 4.0% Trisodium Citratedihydrate (99.0% concentrate, alkaline to make pH 5.5); 94.4% Deionized Water. For example, about 1.5 kg of the preparation is used per about 30 kg of gloves. Each glove weighs, for example, about 6-8 grams, depending, for example, on the size of the glove.

Coated gloves according to the specific embodiment just discussed is prepared, for example, by a process that includes: remove excessive powder from gloves; arranging gloves inside out; chlorinating the gloves; post-process leaching the gloves in hot water; post-leaching in water; removing excessive water (using centrifuge); initial drying; applying (e.g., spraying) the preparation onto loose gloves; second drying; arranging gloves inside in; final drying; cooling; inspecting gloves; and packaging gloves. Some steps of the process may be omitted. Processes are discussed below in further detail.

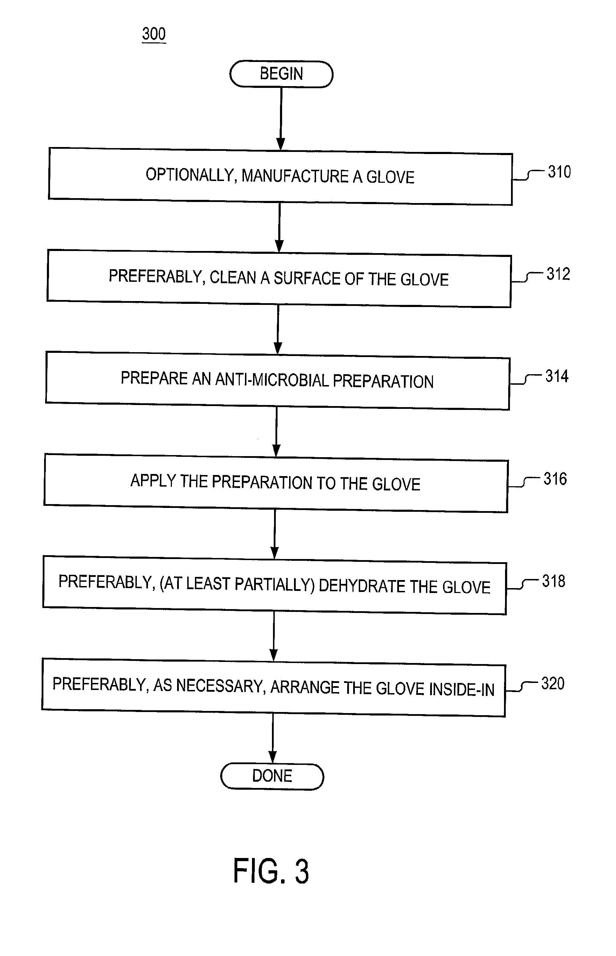

FIG. 3 is a flow diagram that illustrates a process 300, according to some embodiments of the present invention, for making a coated elastomeric flexible article. FIG. 3 is labeled for embodiments in which the elastomeric flexible article is a glove, but FIG. 3 can also be interpreted more generally for non-glove articles.

As shown, the process 300 includes preparing (314) a preparation, for example, any preparation as discussed in the present document. Further, the process 300 includes applying (316) the preparation to a surface of an elastomeric flexible article. Some embodiments of the process 300 include only the steps 314 and 316. However, other embodiments of the process 300 further include some or all of optional steps 310, 312, 318, and 320.

In the preferred embodiment of the process 300, the preparation is then dehydrated (318) at least partially, and preferably at least substantially or fully onto the elastomeric flexible article. Further, as necessary, the elastomeric flexible article is arranged (320) into a final configuration that is inside-in. For example, in an embodiment for making coated gloves, the applying step 316 was preferably onto gloves arranged inside-out, such that the surface that will touch a hand while worn is on the outside. For such a embodiment for making coated gloves, the step 318 is preferably taken to arrange the gloves inside-in.

The step 316 of applying the preparation probably is taken with a clean elastomeric flexible article. Thus, the step 316 is preferably preceded by cleaning (312) the surface of the elastomeric flexible article onto which the preparation will be applied. For increased efficiency, the process 300 is an integrated manufacturing process that includes manufacturing (310) the underlying elastomeric flexible article itself.

The process 300 is preferably operated at a factory where coated elastomeric flexible articles are made in great numbers, for example, at least thousands in each manufacturing session. After their manufacture at the factory, the manufactured gloves are preferably packaged in a form suitable for distribution to end owners and end users via a commercial distribution channel, for example, via wholesalers and other distributors.

As mentioned above, the preparation is preferably at least substantially dehydrated on the article. Accordingly, as the preparation dehydrates, the proportion of ingredients in the preparation will change. Put another way, the preparation as discussed may be diluted excessively by a volatile substance, e.g., water or the like, and then the volatile substance may be allowed to evaporate, at least partially. Furthermore, repeated applications of the preparation and multiple (at least partial) evaporations/dehydrations may be used, for example, to compensate for using excessively diluted preparation. Nevertheless, even if a preparation is excessively diluted, at some time during its evaporation/dehydration process, the preparation will no longer be excessively diluted, relative to the present description, and the proportions of its ingredients can be considered.

Manufacturing processes for treated elastomeric flexible articles may be explained by discussing processes for making treated gloves, with an understanding that the processes may be used to make non-glove treated elastomeric flexible articles as well. According to some embodiments of the present invention, a process for manufacturing a treated elastomeric flexible article is based on any process discussed in either or both of the following two references, which are hereby incorporated in their entirety for all purposes and which are commonly-owned with the present invention: U.S. Patent No. 6,274,154, entitled "Aloe Vera Glove and Manufacturing Method", or U.S. Patent Application No. 09/938,715, Publication Document Number 20020025335, entitled "Aloe Vera Glove and Manufacturing Method". Some embodiments of the present invention are any process as discussed in the two just-mentioned references, but instead of using an "Aloe Vera solution" or the like to coat an article (e.g., glove) as discussed in the two references, a preparation as discussed in the present document is prepared and used to coat an article (e.g., glove).

Some embodiments of the present invention, for making elastomeric flexible articles, will now be discussed. These embodiments include processes based on processes discussed in the just-mentioned two references.

According to an embodiment of the present invention, a method of manufacturing gloves includes treating a commercially available disposable glove to eliminate residue powders, soluble substances, and microorganisms, turning the glove inside out, dipping it into a preparation (any discussed in the present document) and heating the glove to cause water to evaporate.

A glove is preferably first treated with a chlorine solution or chlorine gas. Chlorine solution can help to sterilize the gloves, to wash off powders, and most importantly for natural latex gloves, to dissolve residual proteins that could potentially trigger severe allergic reactions among repeat users. After the outside surface of the glove is treated with the chlorine solution, it is turned inside out, and the glove is again treated with the chlorine solution. The residue chlorine is neutralized by using ammonia and the gloves are then dried.

The preparation will then be prepared, as discussed above. To associate the preparation with the surface of the glove, the preparation can be sprayed onto the surface of the glove. Alternatively, the glove can be immersed into the preparation. The latter method is preferred because it creates a complete and even distribution of the preparation.

In one preferred embodiment, the dipping process is accomplished by grouping a number of gloves in a batch to achieve higher manufacturing efficiency. The gloves are immersed in the preparation for at least 10 minutes to allow adequate absorbency.

The preparation is attached to the surface of the glove through a controlled dehydration process. The water in the preparation solution is caused to evaporate through heating. Although a higher temperature will cause water to evaporate quicker, excess heat may damage the gloves. For example, gloves exposed to excessive heat of over 70°C may turn brownish and become brittle. To shorten the heat exposure time, a heating oven is preheated to about 45°C before the gloves are introduced. The oven has a temperature control mechanism to maintain a maximum temperature. In a preferred embodiment the maximum temperature is set at approximately 65°C and the heating process lasts from about 35 to 40 minutes. The dehydration process provides an affiliation force so that the preparation can remain associated with the glove surface for an extensive period of time.

Even distribution of the preparation on the glove surface maximizes effectiveness and minimizes contact between the skin and the glove's composite material. Stationary drying is not preferred because the preparation solution tends to flow in the direction of the force of gravity. In a preferred embodiment the heating oven has a device to tumble during the heating to make the preparation distribute evenly on the glove surface and to form a uniform coating.

Afterward the gloves are cooled to room temperature. The gloves are then inverted so that the surface with the preparation faces inside.

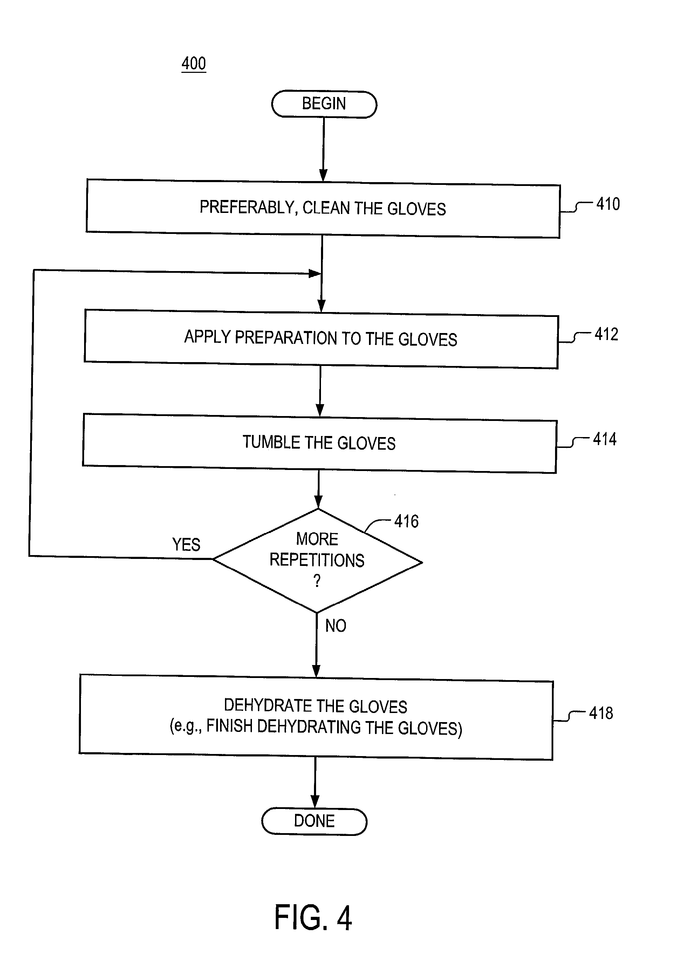

FIG. 4 is a flow diagram that illustrates a method 400 for manufacturing treated gloves, for example, using spraying, according to an embodiment of the present invention, using any preparation as discussed in the present document. The application of the preparation to gloves preferably begins with gloves that are clean and free of protein residue, powder, or other surface contaminants. Therefore, the method 400 preferably begins with a step 410 of cleaning the gloves to remove such contaminants. Next, the preparation is applied to the gloves (step 412), preferably by spraying a batch of clean loose gloves that are arranged inside out. The gloves are tumbled (step 414) so that more gloves become better exposed to current or future applying of the preparation. Preferably, the tumbling of the gloves in the step 414 occurs, or continues to occur, after the spraying of the preparation in the step 412 has already stopped. The steps 412 and 414 are then preferably repeated for a desired number of iterations (as shown by decision box 416 in FIG. 4). After the last iteration of the step 412 of applying the preparation, the gloves are dried (step 418, or the step 418 and the last iteration of the step 414).

The optional (but preferred) step 410 of cleaning the gloves of surface contaminants can be performed using any competent technique (including any conventional technique). For example, as discussed above, a chlorine solution may be used, and the chlorine solution itself is preferably neutralized and cleaned away at the end of the cleaning step. Cleaning items such as gloves of surface contaminants, for example, using chlorine solution, is a known technology, and the specifics of such cleaning would be readily apparent, depending on the particular type of cleaning equipment being used. For example, for a sufficiently large commercial chlorine washer, a batch of about 3000 to 4000 gloves may be washed using any conventional cycle, for example, a cycle of about 20 to 30 minutes, say, about 23 minutes. Optionally, for extra assurance of cleanliness, the batch of gloves may be further rinsed with water, preferably in a separate commercial washing tank, for example first with hot water and then with cold (e.g., room-temperature) water for any desired amount of time, for example, about 20 to 30 minutes or more. For the method 400, the water is preferably drained well from the gloves. For example, the gloves may be spun dry in the commercial washing tank in conventional manner.

Preferably, the steps 412, 414, and 418 are all performed within a commercial heat tumble dryer, for example, as follows. After the optional water bath at the end of the optional cleaning step 410, the gloves are removed from the water bath and dumped into the heat tumble dryer. The dryer then starts tumbling the gloves. Preferably, the tumbling is accompanied by heating of the gloves by hot air and continues until the gloves are dry or mostly dry. Then, a spray nozzle configured to spray the preparation as a fine mist starts spraying the preparation onto the gloves in the dryer. During the spraying, the tumbling may either continue or may continue at a slower pace or may be stopped, and heating of the air may be continued or reduced or stopped. Depending on the level of integration between the spray nozzle and the dryer, the door of the dryer may be opened to allow access to the spray nozzle during spraying. After a period of spraying, the spraying stops and the tumbling continues, or resumes, preferably accompanied by resumed, or continued, heating of the air. The spraying and tumbling are repeated for several iterations. After the last iteration of spraying, the gloves are dried, preferably by tumbling with heating until the gloves are dry. The number and durations of iterations and the amount of solution to use should be chosen to be sufficient, given the particular dryer and spray nozzle configuration, to leave at least a desired minimum thickness, and/or no more than a desired maximum thickness, of dehydrated preparation on substantially every glove.

For example, for a batch of about 3000 gloves, two kilograms of preparation may be sprayed in about 4 or 5 spray iterations, with the spray iterations spaced about 2 to 5 minutes apart, and with each spray iteration's having a spray duration of about 30 to 90 seconds in a dryer that is the oven discussed above (i.e., one that is limited to a maximum temperature of about 65°C (preferred) or less than about 80°C). As shown in FIG. 4, each spray iteration is preferably followed by a tumbling iteration. The final iteration of tumbling is of sufficient duration to dry the gloves and especially should include heating. For example, the final iteration of tumbling may be chosen so that the total duration of tumbling and heating gloves having preparation over all the steps is about 35 to 40 minutes.

Preferably, the method 400 is performed and completed using only two or only three holding containers in which washing, spraying, or tumbling are actually performed. If two containers are used, they would be the chlorine washer and the heat tumble dryer. If three containers are used, they would be the chlorine washer, the water washer, and the heat tumble dryer.

FIG. 4 can also serve as a flow diagram for the earlier-discussed embodiment of the present invention that is a method that uses immersion (e.g., dipping) to apply the preparation. If FIG. 4 is interpreted to describe the method that uses immersion, then preferably the decision box 416 reflects having only a single iteration of immersing (the step 412), and the box for step 414 can be interpreted to refer to agitation of the immersion tank, for example, in the manner of a washing machine. After the immersion (e.g., the Steps 412 and 414), the step 418 refers to tumble drying, as has been discussed earlier. If FIG. 4 is used to describe the immersion method, then preferably an extra holding container would be used, namely, an immersion tank that contains the preparation. Thus, if the method 400 is embodied so as to use immersion, then the method 400 is preferably performed and completed using only three or only four holding containers in which washing, immersion, or tumbling are actually performed. If three containers are used, they would be the chlorine washer, the immersion tank for preparation, and the heat tumble dryer. If four containers are used, they would be the chlorine washer, the washer for water, the immersion tank for preparation, and the heat tumble dryer.

In another embodiment of the present invention, a method for manufacturing treated gloves is integrated with, and/or includes, the manufacturing of the underlying preparation-free gloves themselves. This other embodiment is especially preferred for produce large quantities of treated gloves efficiently.

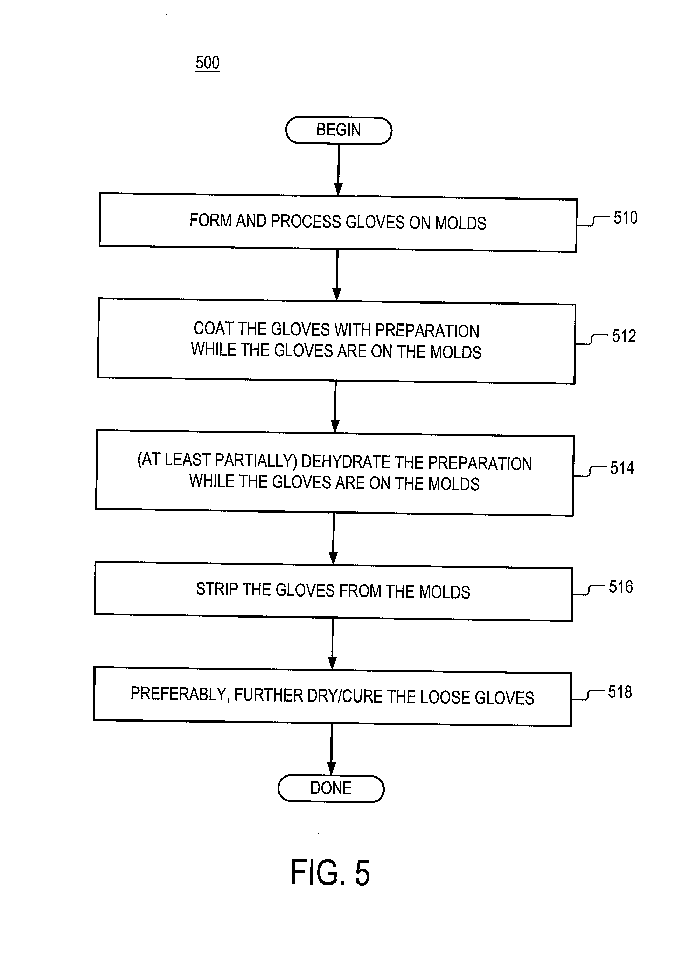

FIG. 5 is a flow diagram that illustrates a method 500, according to an embodiment of the present invention. The method 500 is a method for manufacturing treated gloves that is integrated with, and includes, the manufacturing of the underlying preparation-free gloves themselves. Preferably, the method 500 is fully automated within a production line. In a step 510, gloves are formed on molds using any competent technique, for example, using conventional processes. The forming of gloves on molds is a conventional art and is well known. For example, each mold is shaped to be at least reminiscent of a hand such that the resulting gloves will fit hands. The forming and formed gloves undergo processing on the molds in the step 510 using, for example, conventional processing.

In a step 512, a preparation, for example, any preparation discussed earlier, is applied to the gloves while the gloves are still on the form. The application of the preparation can be via any competent technique, for example, spraying, immersing, pouring, overfilling, dipping, and the like, (which are not mutually exclusive techniques). In a step 514, the preparation that coats the gloves undergoes at least partial, and preferably full or at least substantial, dehydration. Next, in a step 516, the gloves are removed from the molds. Preferably, after removal from the molds, the gloves are further dried and cured by heat, in a step 518.

In the step 510, the gloves formed on the mold are preferably considered to be inside out such that that the interior of each glove, as later to be worn on the hand, faces outward. The gloves are formed and processed using whatever technique is competent to produce a glove of the desired material. The preferred material is natural rubber latex. After a glove is formed, while on the mold, the later hand-facing surface of the glove is preferably made safer, and/or easier to slide during donning, for later contact with hands, either by cleaning off any residual proteins, chemicals, and the like, for example, using chlorine, or by coating the surface with a thin insulating layer that will attempt to insulate the hand from contact with the residual proteins, chemicals, and the like during wearing of the glove. By being cleaned, the glove is likely to be slicker and easier to slide over skin during donning, especially if the glove is made of natural rubber latex. Similarly, the insulating layer is preferably made of a substance that is more slippery than the underlying glove. For example, even if the glove is a vinyl glove of a type that is not made significantly safer or more slippery by cleaning, it may still be coated with an insulating layer to increase its slipperyness and thereby be made easier to don. The insulating layer is, for example, a polymer layer, for example, of silicone or polyurethane,

In the step 512, preparation, such as has already been described, is applied to the gloves while the gloves are still on the molds, either by dipping or by spraying. If spraying is used, it should be thorough enough so as to leave a desired amount of solution on the gloves' inside out surfaces, for example, an amount comparable to that which would be obtained from dipping.

In the step 514, the gloves undergo at least partial, and preferably full or at least substantial, dehydration. For example, fanned heated air may be blown across the gloves on the molds. For natural rubber latex gloves, especially, the air is preferably not more than about 80°C, and even more preferably, the air is not more than about 65°C. Preferably, the preparation is sufficiently dried to provide sufficient adhesion between the preparation and the glove so that the coated glove can withstand the next step 516.

In the step 516, the gloves are stripped from the molds.

In the optional step 518, the loose gloves are further cured, and their coatings are even further dehydrated by heat, for example, in a dryer as has been discussed earlier.

The forming and processing of gloves on molds in the step 510, in one example, includes, on an automatic production line: cleaning porcelain formers (molds) using hot water (for example, about 40°C to 100°C); drying the porcelain formers in hot air (for example, at about 40°C to 100°C); dipping the formers in coagulant (for example, at about 40°C to 70°C); drying the coagulant on the formers in hot air (for example, at about 35°C to 140°C); dipping the coagulant-coated formers in latex (for example, at about 25°C to 45°C); curing the latex on the formers in hot air (for example, at about 60°C to 140°C); leaching the gloves on the formers; beading the edge of the gloves on the formers; and then making the glove surfaces safer, and easier to don, for later contact with hands, either by cleaning or by coating the surface, as discussed above. If cleaning is used in the making-safer/making-easier-to-don step, then the forming and processing further includes: further curing (for example, at about 80°C to 140°C); rinsing with cold water (for example, at no more than room temperature); chlorination (for example, at no more than about 30°C); preferably preceded by further rinsing with cold water (for example, at no more than room temperature); neutralization; further rinsing (for example, with hot followed by cold water); and dehydration and further curing in hot air. Alternatively, if coating is used in the making-safer/making-easier-to-don step, then the forming and processing further includes: drying in hot air (for example, at about 80°C to 150°C); coating with polymer (for example, at no more than about 45°C); and further drying and curing in hot air (for example, at about 80°C to 150°C).

In addition to the preferred natural rubber latex, the present invention may be embodied as preparation-coated gloves of acrylonitrile, polyvinyl chloride, polyurethane, chloroprene, neoprene, butadiene, or the like, and their manufacturer.

Further, in addition to the specific preparations discussed in the present document, or an Aloe Vera solution as discussed in the two references incorporated above (U.S. Patent No. 6,274,154 or U.S. Patent Application No. 09/938,715), the present invention may be embodied to alternatively or additionally use any other substance (e.g., any preparation) that can be dried onto the inside of a glove and that, in the dry form, is mixed with moisture that consists only of perspiration from a hand during wearing of the glove and is beneficial to the hand.

Throughout the description and drawings, example embodiments, for example, products and methods, are given with reference to specific embodiments and configurations. However, the present invention is not limited to those specific embodiments or configurations. It will be appreciated by those of ordinary skill in the art that the present invention can be embodied in other specific forms without departing from the spirit and scope of the present invention.

For example, although glove embodiments are illustrated in FIGS. 1 and 2, any other article or form that contacts skin may also embody the present invention. For example, the present invention may be embodied as elastomeric flexible peels, articles, wraps, and (other) medical devices. Similarly, the composition and application of the preparation may be varied without departing from the spirit and scope of the present invention. For example, various different preparations may be utilized to obtain an ultimate final elastomeric flexible article, for example, a glove, that has characteristics as described within the present document. For example, the formulations of the preparation may be varied in order to have a thicker or thinner coating, as desired to control comfort in use, dexterity, sense of feel, or protection. Still other changes would be apparent.

The scope of the invention is not limited merely to the specific example embodiments or configurations of the foregoing description, but rather is indicated by the appended claims. All changes that come within the meaning and range of equivalents within the claims are intended to be understood as being embraced within the scope of the claims.

## Claims

1. An article comprising:
a disposable protective glove, the disposable protective glove having an interior surface; and
a preparation disposed on the interior surface of the disposable protective glove, wherein the preparation includes an anti-microbial substance, and wherein the preparation includes a buffer that helps resist change in pH during wearing of the disposable protective glove.

2. The article according to claim 1, wherein the anti-microbial substance is acidic during a period when the protective glove is worn, and wherein acidity of the anti-microbial substance contributes substantially to anti-microbial properties of the anti-microbial substance.

3. The article according to claim 2, wherein the preparation has pH within a range of 4.5 to 5.8 during a period in which the preparation is moist.

4. The article according to claim 2, wherein the anti-microbial substance includes an alpha-hydroxy acid.

5. The article according to claim 1, wherein the preparation has been dehydrated onto the interior surface of the protective glove.

6. The article according to claim 5, wherein the anti-microbial substance is to be activated by moisture from hand perspiration during wearing of the protective glove.

7. The article according to claim 1, wherein the buffer includes a weak organic acid and a conjugate base of the weak organic acid.

8. The article according to claim 7, wherein the buffer includes an amino acid and an amino acid salt.

9. The article according to claim 1, wherein the anti-microbial substance includes an acid that exists naturally in an edible plant.

10. The article according to claim 1, configured wherein, after the article, in finished form as would be donned by an end user, has been placed in a chamber at over 30 degrees Celsius and 100% humidity and at least standard pressure for at least 30 minutes, the preparation has pH within the range of 3.8 to 6.0.

11. The article according to claim 1, wherein the preparation does not include antiperspirant.

12. The article according to claim 1, wherein the disposable protective glove is without any layer of porous material

13. An article comprising:
a disposable protective glove, the disposable protective glove having an interior surface; and
a preparation disposed, during factory production of the article, on the interior surface of the disposable protective glove, wherein the preparation includes an anti-bacterial substance, and wherein the preparation includes a buffer that helps resist change in pH during wearing of the disposable protective glove.

14. The article according to claim 13, wherein the buffer includes a quartenized phosphate ester.

15. The article according to claim 13, wherein the anti-bacterial substance includes acid that exists naturally in an edible plant, and the preparation includes substantially no anti-bacterial substance other than the acid that exists naturally in an edible plant.

16. The article according to claim 13, wherein the anti-bacterial substance includes an acid selected from the group consisting of an aliphatic acid, an aromatic acid, salicylic acid, aromatic, aliphatic components, alpha-hydroxy acids, glycolic acid, citric acid, lactic acid, tartaric acid, malic acid, hydroxycaprylic acid, mandelic acid, leucic acid, azelaic acid, ethylglycolic acid, beta-hydroxy acids, salicylic acid, beta-hydroxypropionic acid and beta-hydroxybutyric acid, monocarboxylic acid, dicarboxylic acid, polycarboxylic acid, glucuronic acid, glutamic acid, allantoin galacturonic acid, allantoin glycyrrhetinic acid, allantoin polygalacturonic acid, animal collagen amino acids, animal elastin amino acids, animal keratin amino acids, aspartic acid, folic acid, hyaluronic acid, linoleic acid, linolenic acid, orotic acid, palmitoyl animal collagen amino acids, ribonucleic acid, silk amino acid, uric acid, urocanic acid, dilinoleic acid, trilinoleic acid, alanine, 6-aminocaproic acid, arginine, asparagine, carbocysteine, cysteine, cystine, glycine, histidine, hydroxyproline, isoleucine, leucine, lysine, methionine, phenylalanine, proline, serine, threonine, tryptophan, tyrosine, biotin, o-cresotic acid, glycyrrhetinic acid, glycyrrihizic acid, lanolin acid, and mixtures thereof and carboxylic acids, amino acids, or an organic acid having an ionizable hydrogenated atom.

17. The article according to claim 13, wherein the preparation was disposed onto the disposable examination glove not in powder form.

18. The article according to claim 13, wherein the preparation further includes a skin soothing substance.

19. The article according to claim 13, wherein the preparation includes a thickener.

20. A protective glove comprising:
only a single layer of a fluid-impermeable flexible material having an inner surface and forming a cavity to receive a hand; and
an anti-microbial preparation coated on the inner surface, wherein no aloe vera is coated on the inner surface, and wherein the anti-microbial preparation was factory pre-coated onto the inner surface.
